# EUROPÄISCHE PATENTSCHRIFT

(11) **EP 3 760 273 B1**
(45) Veröffentlichungstag und Bekanntmachung des Hinweises auf die Patenterteilung: **23.04.2025**
(21) Anmeldenummer: 19183995.0
(22) Anmeldetag: 02.07.2019
(51) Int. Cl.: A61M 39/02

(54) **IMPLANTIERBARE HAUTDURCHLASSVORRICHTUNG**
IMPLANTABLE SKIN PENETRATION DEVICE
DISPOSITIF TRANSCUTANÉ POUVANT ÊTRE IMPLANTÉ

(43) Veröffentlichungstag der Anmeldung: 06.01.2021
(73) Patentinhaber: SecureCell AG, 8902 Urdorf (CH)
(72) Erfinder: SCHÜTZ, Daniel, 4912 Aarwangen (CH)
(74) Vertreter: Schmauder & Partner AG Patent- & Markenanwälte VSP

(56) Entgegenhaltungen:
- US-A1- 2012 053 673
- US-A1- 2013 072 847
- US-A1- 2016 279 403
- US-B1- 6 459 917

## Beschreibung

### Technisches Gebiet

Die Erfindung betrifft eine implantierbare Hautdurchlassvorrichtung sowie eine damit ausgestattete Vorrichtung zur Zufuhr und/oder Entnahme von Flüssigkeit an einem Patienten.

### Stand der Technik

Bei Patienten mit Bedarf für wiederholte oder sogar ständige Zuführung von Medikamenten und/oder Entnahme von Körperflüssigkeit werden entsprechende Zugänge, auch "Ports" genannt, angelegt. Derartige Hautdurchlassvorrichtungen umfassen einen Portkörper mit einer von aussen zugänglichen Portkammer, welche in der Regel mit einem im Körperinneren befindlichen Katheterschlauch verbunden ist. Der Zugang zur Portkammer kann auf unterschiedliche Arten erfolgen, insbesondere durch Anstechen. Sehr gebräuchlich sind subkutan implantierte Ports wie beispielsweise in der US 5306255 A beschrieben. Der entsprechende Portkörper liegt dabei vollkommen unter der Haut und ist in Richtung zur Hautoberfläche durch eine Durchstechmembran oder durch eine speziell konfigurierte Anstecheinheit abgeschlossen.

Bei perkutan implantierten Ports wird die Portkammer ebenfalls unter die Haut eingesetzt, aber es verbleibt ein Eintrittssegment, das durch die Haut hindurch nach aussen reicht. Eine derartige perkutane Hautdurchlassvorrichtung ist in der EP 0867197 A2 beschrieben. Im Vergleich zu subkutanen Vorrichtungen ergibt sich der Vorteil eines sicheren und zuverlässigen Zugangs ohne Notwendigkeit eines ständig wiederholten Einstechens in eine lokal begrenzte Hautregion.

Eine gattungsgemässe Hautdurchlassvorrichtung ist in US 2013/072847 A1 beschrieben.

### Darstellung der Erfindung

Aufgabe der Erfindung ist es, eine gattungsgemässe Hautdurchlassvorrichtung zu verbessern und deren Nachteile zu überwinden.

Gelöst werden diese Aufgaben durch die im Anspruch 1 definierte Hautdurchlassvorrichtung sowie durch die im Anspruch 13 definierte Zufuhr- und/oder Entnahmevorrichtung.

Die erfindungsgemässe implantierbare Hautdurchlassvorrichtung für die Verbindung eines externen Schlauches mit einem innerhalb des menschlichen oder tierischen Körpers legbaren Katheterschlauch umfasst einen im Wesentlichen zylindrischen Grundkörper mit einem oberen, im implantierten Zustand körperabgewandten Bereich und einem unteren, im implantierten Zustand körperzugewandten Bereich, wobei der Grundkörper in seinem unteren Bereich mit einer zur subkutanen Einlage vorgesehene Unterhautverankerung ausgestattet ist, und wobei obere Anschlussmittel zum Anschluss des externen Schlauches sowie untere Anschlussmittel zum Anschluss des Katheterschlauches vorhanden sind.

Dadurch, dass die Unterhautverankerung aus einer Mehrzahl von am Grundkörper angelenkten Fixierlaschen gebildet ist, wobei die Fixierlaschen von einer radial nach unten eingeklappten Einfahrstellung in eine radial nach aussen ausgeklappte Haltestellung schwenkbar sind, und dass die unteren Anschlussmittel ein in den Grundkörper einlegbares Adapterstück für den Katheterschlauch aufweisen, das mit den Fixierlaschen zusammenwirkt derart, dass es in einer eingelegten unteren Anschlagstellung die Fixierlaschen in der radial nach aussen ausgeklappten Haltestellung arretiert, ergeben sich diverse Vorteile.

Insbesondere wird aufgrund der zunächst schlanken Gestalt bei eingeklappter Einfahrstellung das Implantieren erleichtert, wobei insbesondere ein vergleichsweise geringerer Hautschnitt ausreichend ist. Dennoch wird anschliessend eine hervorragende Fixierwirkung sichergestellt, weil die Fixierlaschen in arretierter, radial ausgeklappter Haltestellung optimal wirken können. Durch die Zusammenwirkung von Adapterstück und Fixierlaschen wird zudem ein besonders einfacher Zusammenbau der Vorrichtung im Zug des Implantiervorganges erzielt.

Aufgrund des kompakten Aufbaus und der Wirkungsweise als perkutane Vorrichtung werden Totvolumina bei der Flüssigkeitsbeförderung im Vergleich zu den herkömmlichen subkutanen Vorrichtungen, welche einen Anstechbecher für eine externe Injektionsnadel benötigen, extrem reduziert. Die Reduktion bzw. Elimination von Totvolumina und damit einhergehenden Vermischungs- bzw. Verdünnungseffekten ermöglicht es, in neuartiger Weise zeitlich variable Fluidikprozesse mit kleinsten Volumina abzubilden.

Im vorliegenden Zusammenhang sind Richtungsangaben im Hinblick auf den implantierten Zustand der Vorrichtung zu verstehen. "Körperabgewandt" ist also im Sinn von "bezogen auf die Hautoberfläche nach aussen weisend" und "körperzugewandt" entsprechend im Sinn von "bezogen auf die Hautoberfläche nach innen weisend" zu verstehen. Der Einfachheit wird gelegentlich auch auf die Begriffe "oben" als gleichbedeutend mit "körperabgewandt" und "unten" als gleichbedeutend mit "körperzugewandt" zurückgegriffen, weil dies den üblicherweise verwendeten zeichnerischen Darstellungen auf dem Gebiet entspricht. Somit könnte ein "oberer Bereich" der Vorrichtung bei gewissen Einbaulagen und Ausrichtungen des Patienten durchaus nach unten, d.h. gegen den Boden zeigen.

Weiterhin gilt, dass geometrische Angaben nicht im streng mathematischen Sinn, sondern in Anbetracht der zweckgemäss anwendbaren Toleranzen zu verstehen sind.

Darüber hinaus versteht sich, dass Dimensionierung, Materialwahl und Materialbearbeitung entsprechend dem vorgesehenen Zweck der Implantation in den menschlichen oder tierischen Körper erfolgen. Als Werkstoff für die strukturellen Komponenten wie Grundkörper und Fixierlaschen kommt insbesondere Titan, alternativ auch Edelstahl (Medizinalstahl) oder gewisse Kunststoffe in Betracht. Die Abmessungen können im gewohnten Rahmen variieren. So wird der Durchmesser des Katheterschlauchs je nach Anwendung im Bereich von 2 bis 5 mm liegen.

Vorteilhafte Ausführungsformen sind in den abhängigen Ansprüchen definiert.

Hauptaufgabe der Fixierlaschen ist das Verhindern von unerwünschten Verschiebungen der implantierten Hautdurchlassvorrichtung. Vorteilhafterweise (Anspruch 2) sind die Fixierlaschen im Wesentlichen O-förmig und mit gerundeten Kanten ausgebildet und liegen in ihrer Einfahrstellung innerhalb eines durch den Grundkörper definierten Umrisses. Durch die mittige Öffnung kann Haut schnell in die Fixierlasche einwachsen und dadurch die erwünschte Funktion bewirken. Die Ausgestaltung ohne scharfe Kanten und ohne vorstehende Strukturen erleichtert das Einlegen der Hautdurchlassvorrichtung mittels eines vergleichsweise kleinen Hautschnittes.

Prinzipiell kann das Adapterstück auf unterschiedliche Arten in seiner unteren Anschlagstellung gehalten werden. Vorzugsweise (Anspruch 3) ist hierfür der Grundkörper im unteren Bereich mit einem Innengewinde ausgestattet, in welches ein unterer Gewindering einschraubbar ist. Besonders vorteilhaft für die Handhabung ist es, wenn der untere Gewindering mit einer Anzahl von inneren Eingriffsnuten für ein entsprechend ausgebildetes Schraubwerkzeug oder Haltewerkzeug ausgestattet ist (Anspruch 4).

Bei einer Ausführungsform (Anspruch 5) ist der Grundkörper in seinem oberen Bereich mit randseitigen Vertiefungen zum Anbringen eines Stützringes ausgestattet ist. Dies erlaubt in vorteilhafter Weise, bei notwendigen Handhabungen wie insbesondere dem Anschliessen des externen Schlauches eine mechanische Stabilisierung zu leisten. Es werden also unerwünschte Zug- und Schiebekräfte wie auch Kippmomente vermieden.

Bei einer vorteilhaften Ausführungsform (Anspruch 6) umfassen die oberen Anschlussmittel ein in den Grundkörper einschraubbares Spannelement, welches einen zur füllenden Aufnahme eines Septumkörpers ausgebildeten Hohlraum aufweist, welcher durch eine untere Bodenfläche begrenzt ist, wobei letztere mindestens eine Durchgangsöffnung aufweist.

Bezüglich der oberen Anschlussmittel ist es vorteilhaft (Anspruch 7), wenn diese einen mit dem externen Schlauch verbindbaren Anschlusskopf umfassen, welcher am Spannelement oberseitig anschliessbar ist. Für die erforderliche Ausrichtung und Fixierung sind Anschlusskopf und Spannelement mit zusammenwirkenden Kopplungselementen ausgestattet. Vorzugsweise (Anspruch 8) umfassen die zusammenwirkenden Kopplungselemente gemäss einem Steckerprinzip eine obere Ausnehmung im Spannelement und einen zugehörigen Vorsprung im Anschlusskopf.

Gemäss einer vorteilhaften Ausführungsform (Anspruch 9) umfasst das Adapterstück der unteren Anschlussmittel einen Adapterkopf mit einer Basisfläche und einer Ausnehmung zur dichtenden Einlage des Katheterschlauches, wobei zwischen der Ausnehmung und der oberen Basisfläche mindestens ein Durchgangskanal ausgebildet. Im zusammengebauten Zustand der Hautdurchlassvorrichtung ist die Basisfläche zur körperabgewandten, oberen Seite gerichtet und bildet eine Kontaktfläche für ein darüber eingeschraubtes Spannelement. Vorzugsweise (Anspruch 10), ist der Adapterkopf kalottenförmig ausgebildet derart, dass beim Einschieben des Adapterstücks in den Grundkörper bis hin zur unteren Anschlagstellung ein Flächenbereich des Adapterkopfes mit entsprechend geformten Endbereichen der Fixierlaschen zusammenwirkt und diese in die radial ausgeklappte Haltestellung schwenkt. Es handelt sich hierbei also um eine kalottenförmige Struktur, die im zusammengebauten Zustand der Hautdurchlassvorrichtung zur körperzugewandten, unteren Seite gerichtet ist. Besonders bevorzugt ist zudem (Anspruch 11), dass die Ausnehmung zur dichtenden Einlage des Katheterschlauches seitlich am Adapterkopf angeordnet ist. Mit anderen Worten wird der Katheterschlauch im Wesentlichen parallel zur Hautoberfläche unterhalb derselben weggeführt.

Gemäss einer besonders bevorzugten Ausführungsform (Anspruch 12) ist der Durchgangskanal im Adapterkopf durch mindestens eine, vorzugsweise drei Kanüle(n) gebildet ist, wobei jede Kanüle wie folgt konfiguriert ist:
- ein unteres Kanülenende ragt aus der seitlichen Ausnehmung hervor,
- ein daran anschliessender mittiger Kanülenbereich ist bogenförmig durch den Adapterkopf geführt,
- ein daran anschliessendes oberes Kanülenende ragt aus der Basisfläche hervor. Jedes seitlich herausragende untere Kanülenende bildet einen Anschluss für einen zugeordneten Innenkanal eines an die Vorrichtung anzusetzenden Katheterschlauches.

Jedes in körperabgewandter Richtung hervor ragende obere Kanülenende ist dazu vorgesehen, in einen zugeordneten Septumkanal eines darüber angebrachten Septumkörpers zu reichen.

In einer Grundausführung umfasst die Hautdurchlassvorrichtung einen einzigen Flüssigkeitskanal. Für manche Anwendungen ist es von Vorteil, über drei Innenkanäle zu verfügen, wovon je ein Kanal für die Zuführung bzw. Abführung von Flüssigkeit dient, während ein dritter Kanal zur Aufnahme eines Führungsdrahtes vorgesehen ist.

Gemäss einem weiteren Aspekt der Erfindung (Anspruch 13) umfasst eine Vorrichtung zur Zufuhr und/oder Entnahme von Flüssigkeit an einem Patienten eine erfindungsgemässe Hautdurchlassvorrichtung, einen daran angeschlossenen Katheterschlauch sowie einen daran angeschlossenen externen Schlauch, welcher an eine entsprechende Prozesseinheit anschliessbar ist. Bei der Flüssigkeit kann es sich insbesondere um entnommene Körperflüssigkeit, namentlich Patientenblut, oder um eine zugeführte Wirkstofflösung handeln. Ferner kann es je nach Prozedur zumindest zu gewissen Zeitpunkten erforderlich sein, eine Spüllösung durch einen der Flüssigkeitskanäle oder ggf. sogar durch beide Flüssigkeitskanäle zu befördern.

Besonders vorteilhaft (Anspruch 14) ist es dabei, wenn die oberen Anschlussmittel ein in den Grundkörper eingeschraubtes Spannelement umfassen, in dessen Hohlraum ein Septumkörper füllend eingesetzt ist, wobei der Septumkörper mindestens einen durchgängigen Septumkanal aufweist, in welchen einerseits ein oberes Kanülenende des Adapterstücks und andererseits ein mit dem externen Schlauch kommunizierendes unteres Röhrchenende des Anschlusskopfes eingesteckt sind.

### Kurze Beschreibung der Zeichnungen

Ausführungsbeispiele der Erfindung werden nachfolgend anhand der Zeichnungen näher beschrieben, dabei zeigen:
- Fig. 1: eine Hautdurchlassvorrichtung, in einer vertikalen Schnittdarstellung;
- Fig. 2: die Hautdurchlassvorrichtung der Fig. 1, in Draufsicht;
- Fig. 3: die Hautdurchlassvorrichtung der Fig. 1 mit aufgesetztem Anschlusskopf und externem Schlauch, in einer vertikalen Schnittdarstellung;
- Fig. 4: die Hautdurchlassvorrichtung der Fig. 3, in Draufsicht;
- Fig. 5: Bestandteile einer Hautdurchlassvorrichtung, in einer Explosionsdarstellung;
- Fig. 6: einen Grundkörper mit Fixierlaschen in radial eingeklappter Einfahrstellung, in einer perspektivischen Darstellung;
- Fig. 7: den Grundkörper der Fig. 6, unter die Haut gesetzt und mit Fixierlaschen in radial ausgeklappter Haltestellung, in einer vertikalen Schnittdarstellung;
- Fig. 8: den Grundkörper der Fig. 7, mit heraus ragendem Katheterschlauch, in einer vertikalen Schnittdarstellung;
- Fig. 9: den Grundkörper der Fig. 8, mit an dem Katheterschlauch angebrachtem Adapterstück, in einer vertikalen Schnittdarstellung;
- Fig. 10: den Grundkörper der Fig. 9, mit teilweise in dem Grundkörper eingelegtem Adapterstück, in einer vertikalen Schnittdarstellung;
- Fig. 11: den Grundkörper der Fig. 10, mit vollständig in dem Grundkörper eingelegtem Adapterstück und darüber eingeschraubtem Gewindering, in einer perspektivischen Darstellung;
- Fig. 12: den Grundkörper der Fig. 11, mit vollständig in dem Grundkörper eingelegtem Adapterstück und darüber eingeschraubtem Spannelement, in einer perspektivischen Darstellung;
- Fig. 13: eine implantierte Hautdurchlassvorrichtung, in einer perspektivischen Darstellung;
- Fig. 14: die implantierte Hautdurchlassvorrichtung der Fig. 13, mit angesetztem Stützring, in einer perspektivischen Darstellung;
- Fig. 15: die implantierte Hautdurchlassvorrichtung der Fig. 14, mit aufgesetztem Anschlusskopf und externem Schlauch, in einer perspektivischen Darstellung; und
- Fig. 16: die implantierte Hautdurchlassvorrichtung der Fig. 15, nach Entfernen des Stützrings, in einer perspektivischen Darstellung.

### Wege zur Ausführung der Erfindung

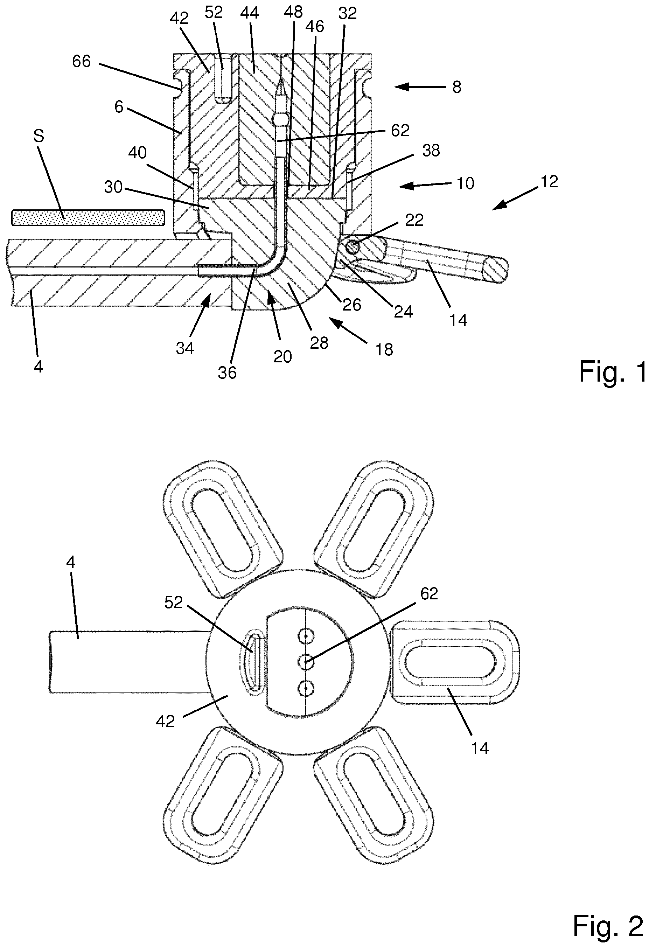

Die Fig. 1 bis 4 zeigen eine implantierbare Hautdurchlassvorrichtung für die Verbindung eines externen Schlauches 2 mit einem innerhalb des menschlichen oder tierischen Körpers legbaren Katheterschlauch 4. Die Vorrichtung umfasst einen im Wesentlichen zylindrischen Grundkörper 6, mit einem oberen, im implantierten Zustand körperabgewandten Bereich 8 und einem unteren, im implantierten Zustand körperzugewandten Bereich 10. Zur Veranschaulichung der Einbaulage in Bezug auf die benachbarte Hautschicht S ist ein kleines Hautsegment in der Fig. 1 dargestellt. Der Grundkörper in seinem unteren Bereich mit einer zur subkutanen Einlage vorgesehene Unterhautverankerung 12 ausgestattet, welche aus einer Mehrzahl von am Grundkörper angelenkten Fixierlaschen 14 gebildet ist. Die Fixierlaschen sind von einer hier nicht dargestellten radial eingeklappten Einfahrstellung E in die hier gezeigte radial ausgeklappte Haltestellung H schwenkbar.

Ausserdem ist die Hautdurchlassvorrichtung mit oberen Anschlussmitteln 16 zum Anschluss des externen Schlauches sowie mit unteren Anschlussmitteln 18 zum Anschluss des Katheterschlauches ausgestattet.

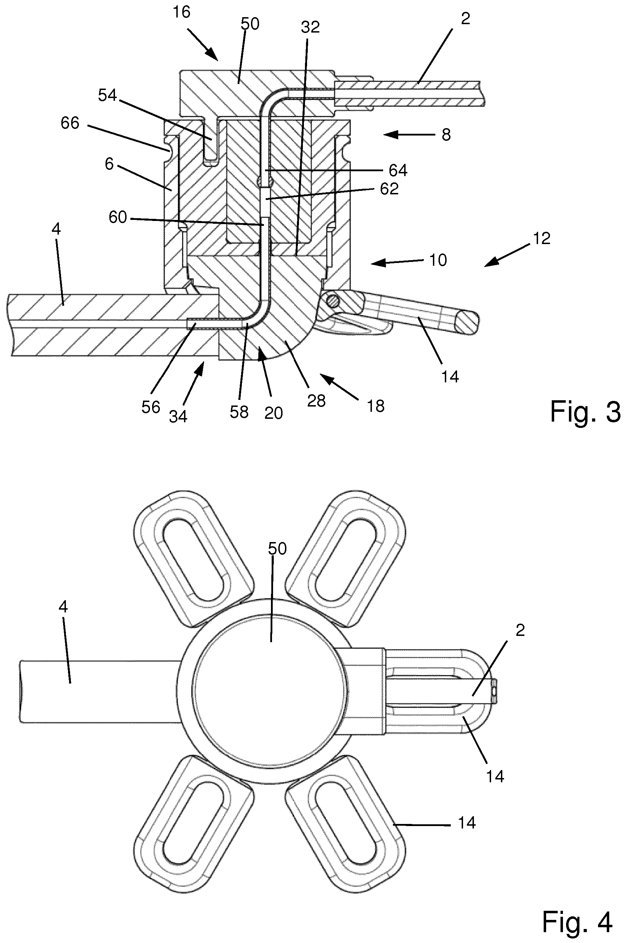

Die unteren Anschlussmittel weisen ein in den Grundkörper einlegbares Adapterstück 20 für den Katheterschlauch auf, das mit den Fixierlaschen zusammenwirkt derart, dass es in der in den Fig. 1 und 3 gezeigten unteren Anschlagstellung die Fixierlaschen in der radial ausgeklappten Haltestellung H fixiert. Zu diesem Zweck ist im gezeigten Beispiel jede Fixierlasche 14 an einer zugeordneten Schwenkachse 22 montiert und weist einen vom Hauptsegment der Lasche abgewandten nockenartigen Endbereich 24 auf. Letzterer liegt an einem entsprechend geformten äusseren Flächenbereich 26 eines kalottenförmigen Adapterkopfes 28 an. So wird durch Absenken des Adapters 20 der Endbereich 24 mit abgesenkt und entsprechend das Hauptsegment der Fixierlasche angehoben und arretiert. Wie ebenfalls aus den Fig. 1 und 3 hervorgeht, weist der Adapterkopf einen flanschartigen Abschnitt 30 mit einer Basisfläche 32 und einer seitlich angeordneten Ausnehmung 34 zur dichtenden Einlage des Katheterschlauches ausgestaltet, wobei zwischen der Ausnehmung und der oberen Basisfläche mindestens ein Durchgangskanal 36 ausgebildet ist.

Im gezeigten Beispiel ist der Grundkörper 6 im unteren Bereich mit einem Innengewinde 38 ausgestattet, in welches ein unterer Gewindering 40 einschraubbar ist, um das Adapterstück 20 in seiner unteren Anschlagstellung zu halten.

Die oberen Anschlussmittel umfassen ein in den Grundkörper einschraubbares Spannelement 42, welches einen zur füllenden Aufnahme eines Septumkörpers 44 ausgebildeten Hohlraum aufweist, der durch eine untere Bodenfläche 46 mit mindestens einer Durchgangsöffnung 48 begrenzt ist. Wie insbesondere aus den Fig. 3 und 4 hervorgeht, umfassen die oberen Anschlussmittel einen mit dem externen Schlauch verbindbaren Anschlusskopf 50, welcher am Spannelement oberseitig anschliessbar ist, wobei der Anschlusskopf und das Spannelement mit zusammenwirkenden Kopplungselementen ausgestattet sind. Im gezeigten Beispiel handelt es sich dabei jeweils um eine obere Ausnehmung 52 im Spannelement und einen zugehörigen Vorsprung 54.

Wie insbesondere in den Fig. 1 bis 3 gezeigt, ist der Durchgangskanal im Adapterkopf 28 durch drei Kanülen gebildet, wobei jede Kanüle wie folgt konfiguriert ist:
- ein unteres Kanülenende 56 ragt aus der seitlichen Ausnehmung 34 hervor,
- ein daran anschliessender mittiger Kanülenbereich 58 ist bogenförmig durch den Adapterkopf geführt,
- ein daran anschliessendes oberes Kanülenende 60 ragt aus der Basisfläche hervor und ist vorzugsweise mit einer Spitze versehen. Dieses obere Kanülenende führt in einen Durchgangskanal 62 des darüber liegenden Septumkörpers 44.

In den Fig. 3 und 4 ist die Hautdurchlassvorrichtung in einsatzbereitem Zustand dargestellt, wobei ein mit dem externen Schlauch 2 kommunizierendes unteres Röhrchenende 64 des Anschlusskopfes 50 in den Durchgangskanal 62 des Septumkörpers 44 eingesteckt ist.

Aus einer Zusammenschau der Fig. 1 und 3 ist ersichtlich, dass das Septum 44 eine innere Bohrung 62 aufweist, welche durch die Kanüle 64 durchstochen und so aufgespreizt wird.

Wie aus den Fig. 2 und 4 hervorgeht, sind die Fixierlaschen im Wesentlichen O-förmig und mit gerundeten Kanten ausgebildet. Weiterhin ist der Grundkörper in seinem oberen Bereich mit randseitigen Vertiefungen 66 zum Anbringen eines Stützringes ausgestattet.

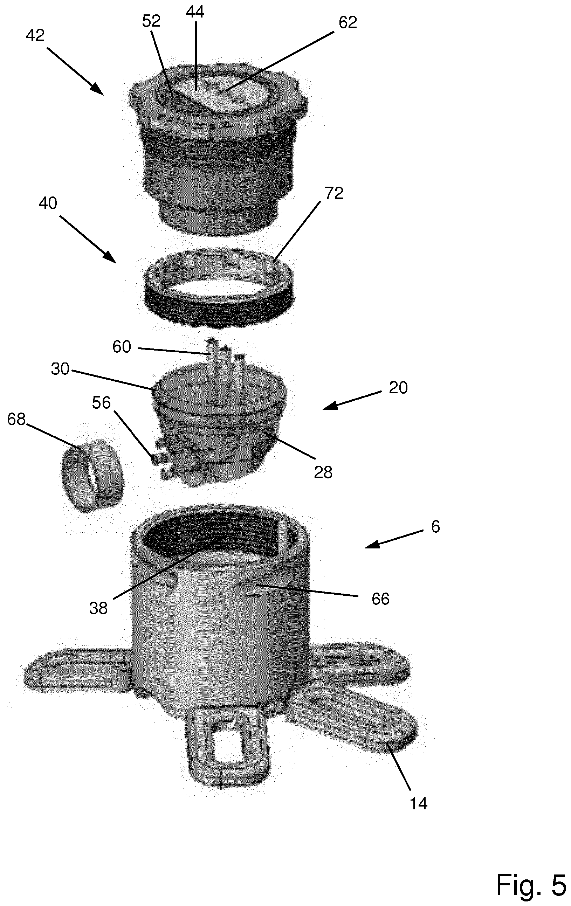

Die Funktionsweise und Benutzung der Hautdurchlassvorrichtung wird in den Fig. 5 bis 16 noch weiter erläutert. Bereits mit den obigen Figuren eingeführte Merkmale sind mit übereinstimmenden Bezugszeichen versehen und werden nur soweit nötig nochmals erwähnt.

Die Fig. 5 zeigt zunächst wichtige Komponenten einer Hautdurchlassvorrichtung, in einer Explosionsdarstellung. Neben den bereits diskutierten Komponenten ist ein Klemmring 68 für den Katheterschlauch dargestellt.

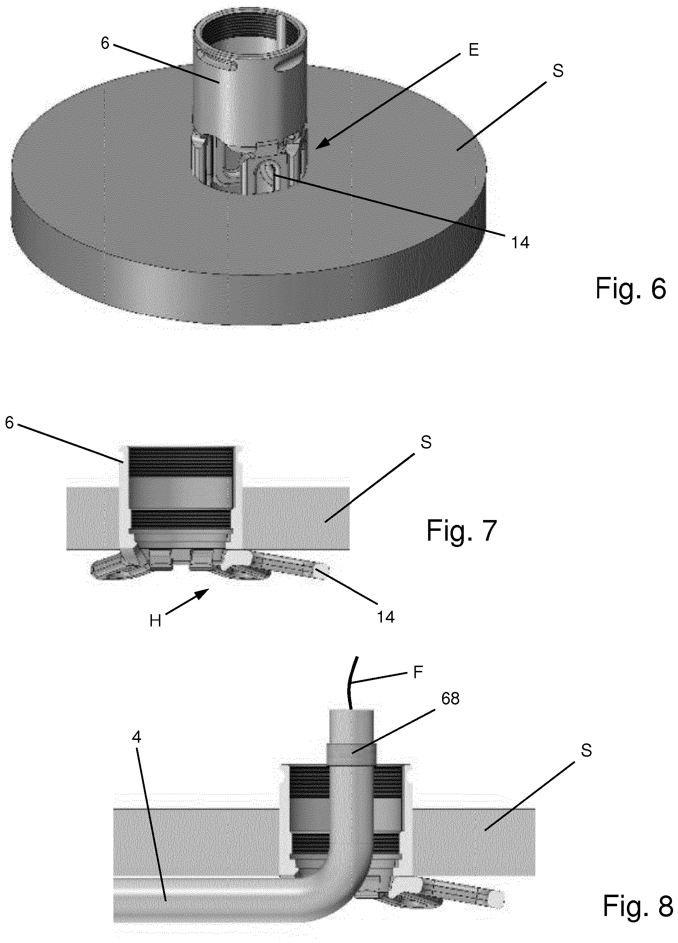

Die Fig. 6 zeigt einen Grundkörper 6 mit Fixierlaschen 14 in radial eingeklappter Einfahrstellung, oberhalb eines schematisch dargestellten Hautbereiches H. Dabei ist ersichtlich, dass die Fixierlaschen in ihrer Einfahrstellung innerhalb eines durch den Grundkörper definierten Umrisses befinden. Dementsprechend muss in der Haut ein vergleichsweise kleines Segment aufgeschnitten werden, was mit einer herkömmlichen Hautdurchlassvorrichtung mit starrer Unterhautverankerung nicht ausreichend wäre. In der Fig. 7 ist sodann die Situation bei Fixierlaschen in radial ausgeklappter Haltestellung ersichtlich.

Im praktischen Operationsablauf wird vor dem Einsetzen der Hautdurchlassvorrichtung in grundsätzlich bekannter Art und Weise der Katheterschlauch 4 eingeführt und an gewünschter Stelle beispielsweise in ein Blutgefäss eingeführt. In der Regel erfolgt dies unter Zuhilfenahme eines Führungsdrahtes F. Dies ist in der Fig. 8 veranschaulicht, wo auch der auf den Katheterschlauch gestülpte Klemmring 68 sichtbar ist.

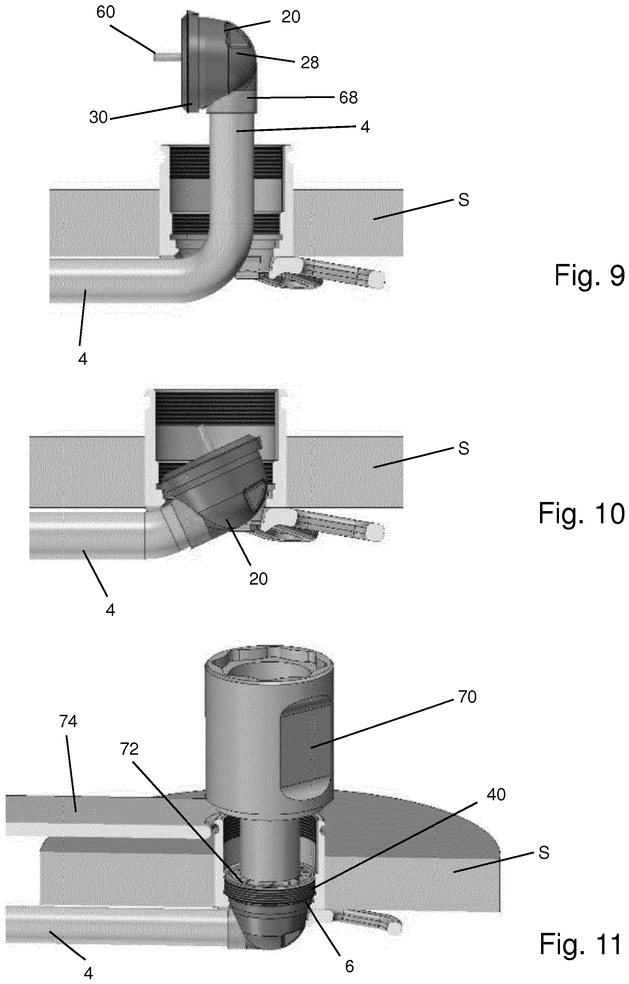

Die Fig. 9 zeigt die Situation nachdem an dem Katheterschlauch ein Adapterstück 20 angebracht und durch Klemmen des Klemmrings 28 befestigt wurde. In der Folge wurde der Katheterschlauch 4 ins Körperinnere gezogen, was in bekannter Weise über eine Schlaufenführung zu einem zweiten Hautschnitt erfolgt. Es ergibt sich danach die in Fig. 10 dargestellte Situation, bei welcher das Adapterstück in dem Grundkörper eingezogen wird.

Die Fig. 11 zeigt sodann den Grundkörper der Fig. 10 mit vollständig eingelegtem Adapterstück 20 und darüber eingeschraubtem Gewindering 40. Ebenfalls dargestellt ist ein Schraubwerkzeug 70, welches mit einem ersten Ende in entsprechend konfigurierte Eingriffsnuten 72 des Gewinderings eingesetzt wird. Darüber hinaus ist ein Haltewerkzeug 74 gezeigt, welches den Grundkörper 6 an den bereits erwähnten randseitigen Vertiefungen 66 ergreift.

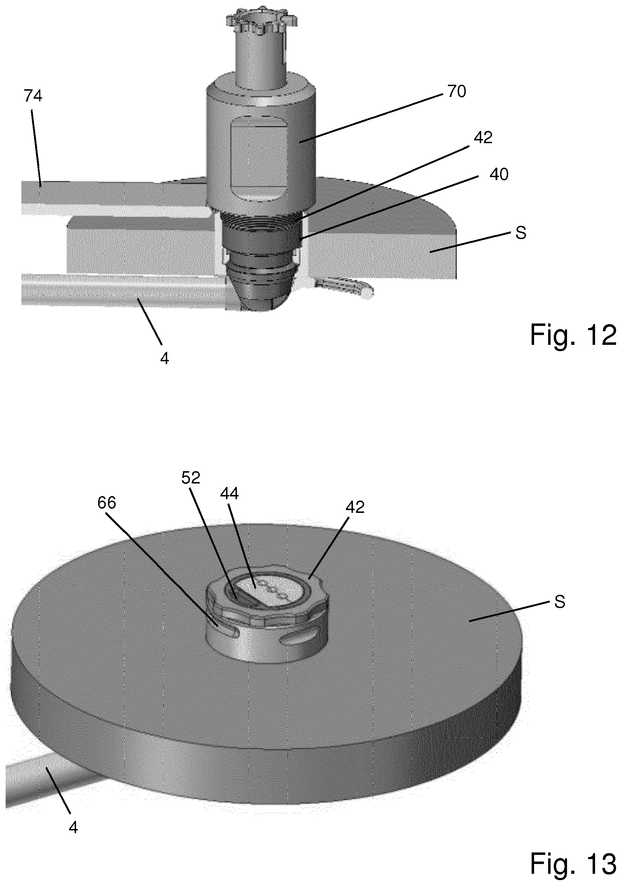

Die Fig. 12 zeigt die Situation mit vollständig in dem Grundkörper eingelegtem Adapterstück 20 und darüber eingeschraubtem Spannelement 42. Hierfür wird vorteilhafterweise wiederum das Schraubwerkzeug 70 verwendet, welches an seinem zweiten Ende wie ein Ringschlüssel konfiguriert ist und damit eine entsprechend geformte obere Randzone des Spannelementes umgreift. Nach Abnahme des Schraubwerkzeugs und des Haltewerkzeugs ergibt sich schliesslich die in Fig. 13 dargestellte Situation.

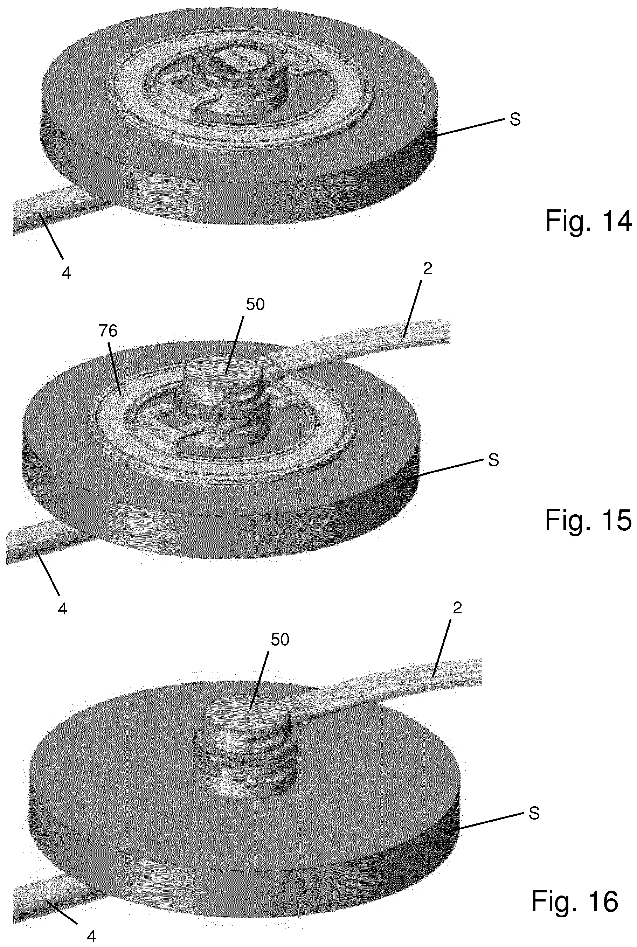

In den Fig. 14 bis 16 ist noch das Anbringen des mit dem externen Schlauch 2 verbundenen Anschlusskopfes 50 dargestellt. Es wird zunächst ein spezifisch konfigurierter Stützring 76 an den bereits erwähnten randseitigen Vertiefungen 66 des Grundkörpers 6 befestigt. Danach wird der Anschlusskopf 50 aufgesetzt und schliesslich der Stützring 76 wieder abgenommen. Damit ist die Hautdurchlassvorrichtung grundsätzlich betriebsbereit.

Die oben beispielhaft beschriebene Hautdurchlassvorrichtung ist für die Zufuhr und/oder Entnahme von Flüssigkeit an einem Patienten in grundsätzlich bekannter Weise an eine hier nicht dargestellte, entsprechende Prozesseinheit anschliessbar.

## Patentansprüche

1. Implantierbare Hautdurchlassvorrichtung für die Verbindung eines externen Schlauches (2) mit einem innerhalb des menschlichen oder tierischen Körpers legbaren Katheterschlauch (4), umfassend einen im Wesentlichen zylindrischen Grundkörper (6), mit einem oberen, im implantierten Zustand körperabgewandten Bereich (8) und einem unteren, im implantierten Zustand körperzugewandten Bereich (10), wobei der Grundkörper in seinem unteren Bereich mit einer zur subkutanen Einlage vorgesehene Unterhautverankerung (12) ausgestattet ist, und wobei obere Anschlussmittel (16) zum Anschluss des externen Schlauches sowie untere Anschlussmittel (18) zum Anschluss des Katheterschlauches vorhanden sind, **dadurch gekennzeichnet,**
**dass** die Unterhautverankerung aus einer Mehrzahl von am Grundkörper angelenkten Fixierlaschen (14) gebildet ist, wobei die Fixierlaschen von einer radial nach unten eingeklappten Einfahrstellung (E) in eine radial nach aussen ausgeklappte Haltestellung (H) schwenkbar sind, und
**dass** die unteren Anschlussmittel ein in den Grundkörper einlegbares Adapterstück (20) für den Katheterschlauch aufweisen, das mit den Fixierlaschen zusammenwirkt derart, dass es in einer eingelegten unteren Anschlagstellung die Fixierlaschen in der radial nach aussen ausgeklappten Haltestellung (H) fixiert.

2. Hautdurchlassvorrichtung nach Anspruch 1, wobei die Fixierlaschen (14) im Wesentlichen O-förmig und mit gerundeten Kanten ausgebildet sind und in ihrer Einfahrstellung (E) innerhalb eines durch den Grundkörper definierten Umrisses liegen.

3. Hautdurchlassvorrichtung nach Anspruch 1 oder 2, wobei der Grundkörper im unteren Bereich mit einem Innengewinde (38) ausgestattet ist, in welches ein unterer Gewindering (40) einschraubbar ist, um das Adapterstück in seiner unteren Anschlagstellung zu halten.

4. Hautdurchlassvorrichtung nach Anspruch 3, wobei der untere Gewindering mit einer Anzahl von inneren Eingriffsnuten (72) für ein entsprechend ausgebildetes Schraubwerkzeug (70) ausgestattet ist.

5. Hautdurchlassvorrichtung nach einem der Ansprüche 1 bis 4, wobei der Grundkörper in seinem oberen Bereich mit randseitigen Vertiefungen (66) zum Anbringen eines Stützringes (76) oder Haltewerkzeugs (74) ausgestattet ist.

6. Hautdurchlassvorrichtung nach einem der Ansprüche 1 bis 5, wobei die oberen Anschlussmittel ein in den Grundkörper einschraubbares Spannelement (42) umfassen, welches einen zur füllenden Aufnahme eines Septumkörpers (44) ausgebildeten Hohlraum aufweist, welcher durch eine untere Bodenfläche (46) mit mindestens einer Durchgangsöffnung (48) begrenzt ist.

7. Hautdurchlassvorrichtung nach Anspruch 6, wobei die oberen Anschlussmittel überdies einen mit dem externen Schlauch verbindbaren Anschlusskopf (50) umfassen, welcher am Spannelement (42) oberseitig anschliessbar ist, wobei der Anschlusskopf und das Spannelement mit zusammenwirkenden Kopplungselementen (52,54) ausgestattet sind.

8. Hautdurchlassvorrichtung nach Anspruch 7, wobei die zusammenwirkenden Kopplungselemente eine obere Ausnehmung (52) im Spannelement und einen zugehörigen Vorsprung (54) im Anschlusskopf umfassen.

9. Hautdurchlassvorrichtung nach einem der Ansprüche 1 bis 8, wobei das Adapterstück (20) der unteren Anschlussmittel einen Adapterkopf (28) mit einer Basisfläche (32) und einer Ausnehmung (34) zur dichtenden Einlage des Katheterschlauches umfasst, wobei zwischen der Ausnehmung und der oberen Basisfläche mindestens ein Durchgangskanal (36) ausgebildet ist.

10. Hautdurchlassvorrichtung nach Anspruch 9, wobei der Adapterkopf (28) kalottenförmig ausgebildet ist derart, dass beim Einschieben des Adapterstücks in den Grundkörper bis hin zur unteren Anschlagstellung ein Flächenbereich (26) des Adapterkopfes mit entsprechend geformten Endbereichen (24) der Fixierlaschen zusammenwirkt und diese in die radial ausgeklappte Haltestellung schwenkt.

11. Hautdurchlassvorrichtung nach Anspruch 10, wobei die Ausnehmung (34) zur dichtenden Einlage des Katheterschlauches (4) seitlich am Adapterkopf (28) angeordnet ist.

12. Hautdurchlassvorrichtung nach Anspruch 11, wobei der Durchgangskanal im Adapterkopf (28) durch mindestens eine, vorzugsweise drei Kanüle(n) gebildet ist, wobei jede Kanüle wie folgt konfiguriert ist:
- ein unteres Kanülenende (56) ragt aus der seitlichen Ausnehmung (34) hervor,
- ein daran anschliessender mittiger Kanülenbereich (58) ist bogenförmig durch den Adapterkopf geführt,
- ein daran anschliessendes oberes Kanülenende (60) ragt aus der Basisfläche hervor.

13. Vorrichtung zur Zufuhr und/oder Entnahme von Flüssigkeit an einem Patienten, umfassend eine Hautdurchlassvorrichtung nach einem der vorangehenden Ansprüche, einen daran angeschlossenen Katheterschlauch (4) sowie einen daran angeschlossenen externen Schlauch (2), welcher an eine entsprechende Prozesseinheit anschliessbar ist.

14. Vorrichtung nach Anspruch 13, wobei die oberen Anschlussmittel ein in den Grundkörper eingeschraubtes Spannelement (42) umfassen, in dessen Hohlraum ein Septumkörper (44) füllend eingesetzt ist, wobei der Septumkörper mindestens einen durchgängigen Septumkanal (62) aufweist, in welchen einerseits ein oberes Kanülenende (60) des Adapterstücks und andererseits ein mit dem externen Schlauch (2) kommunizierendes unteres Röhrchenende (64) des Anschlusskopfes (50) eingesteckt sind.

## Claims

1. An implantable skin piercing device for connecting an external tube (2) to a catheter tube (4) that can be placed inside the human or animal body, comprising: a substantially cylindrical base body (6) with an upper region (8) facing away from the body in the implanted state and a lower region (10) facing the body in the implanted state, wherein the base body is provided in its lower region with a subcutaneous anchorage (12) intended for subcutaneous insertion and wherein upper connection means (16) for connecting the external tube and lower connection means (18) for connecting the catheter tube are available,
**characterized in that**
the subcutaneous anchorage is formed from a plurality of fixing tabs (14) articulated on the base body, and wherein the fixing tabs are pivotable from a radially downwards folded insertion position (E) into a radially outwards folded holding position (H), and
the lower connection means comprise an adapter piece (20) for the catheter tube which can be inserted into the base body and which cooperates with the fixing tabs in such manner that in an inserted lower stop position, it fixes the fixing tabs in the radially outwards folded holding position (H).

2. The skin piercing device according to claim 1, wherein the fixing tabs (14) are formed substantially O-shaped and with rounded edges and, in their insertion position (E), lie within an outline defined by the base body.

3. The skin piercing device according to claim 1 or 2, wherein the base body is provided in the lower region thereof with an internal thread (38), into which a lower threaded ring (40) can be screwed in order to hold the adapter piece in its lower stop position.

4. The skin piercing device according to claim 3, wherein the lower threaded ring is provided with a plurality of internal engagement grooves (72) for a correspondingly shaped screwing tool (70).

5. The skin piercing device according to one of claims 1 to 4, wherein the base body is provided in its upper region with peripheral depressions (66) for attaching a support ring (76) or holding tool (74).

6. The skin piercing device according to one of claims 1 to 5, wherein the upper connection means comprise a tensioning element (42) which can be screwed into the base body and which comprises a cavity formed to receive a septum body (44) in a filling manner, which cavity is delimited by a lower bottom surface (46) with at least one passage opening (48).

7. The skin piercing device according to claim 6, wherein the upper connection means further comprises a connection head (50) which is connectable to the external tube and which is connectable to the tensioning element (42) on the upper side, wherein the connection head and the tensioning element are provided with cooperating coupling elements (52, 54).

8. The skin piercing device according to claim 7, wherein the cooperating coupling elements comprise an upper recess (52) in the tensioning element and a corresponding projection (54) in the connecting head.

9. The skin piercing device according to one of claims 1 to 8, wherein the adapter piece (20) of the lower connection means comprises an adapter head (28) with a base surface (32) and a recess (34) for a sealing insertion of the catheter tube, wherein at least one traversing channel (36) is formed between the recess and the upper base surface.

10. The skin piercing device according to claim 9, wherein the adapter head (28) is formed in the shape of a dome in such manner that when the adapter piece is inserted into the base body up to the lower stop position, a surface region (26) of the adapter head cooperates with correspondingly shaped end regions (24) of the fixing tabs and pivots these into the radially outwards folded holding position.

11. The skin piercing device according to claim 10, wherein the recess (34) for the sealing insertion of the catheter tube (4) is arranged laterally on the adapter head (28).

12. The skin piercing device according to claim 11, wherein the traversing channel in the adapter head (28) is formed by at least one, preferably three, cannula(s), wherein each cannula is configured as follows:
- a lower cannula end (56) protrudes from the lateral recess (34),
- an adjoining central cannula region (58) is guided in an arc-shaped manner through the adapter head,
- an adjoining upper cannula end (60) protrudes from the base surface.

13. A device for supplying and/or withdrawing fluid from a patient, comprising: a skin piercing device according to one of the preceding claims, a catheter tube (4) connected thereto and an external tube (2) connected thereto, which can be connected to a corresponding process unit.

14. The device according to claim 13, wherein the upper connection means comprise a tensioning element (42) which is screwed into the base body, in the cavity of which a septum body (44) is inserted in a filling manner, wherein the septum body comprises at least one continuous septum channel (62), into which, on the one hand, an upper cannula end (60) of the adapter piece and, on the other hand, a lower tube end (64) of the connection head (50) communicating with the external tube (2) are inserted.

## Revendications

1. Dispositif de passage cutané implantable pour connecter un tube externe (2) à un tube cathéter (4) qui peut être placé à l'intérieur du corps humain ou animal, comprenant un corps de base (6) sensiblement cylindrique avec une région supérieure (8) orientée à l'opposé du corps à l'état implanté et une région inférieure (10) orientée vers le corps à l'état implanté, le corps de base étant équipé dans sa région inférieure d'un ancrage sous-cutané (12) destiné à une insertion sous-cutanée, et la région supérieure comprenant des moyens de connexion (16) supérieurs pour connecter le tube externe et des moyens de connexion inférieurs (18) pour connecter le tube cathéter, **caractérisé en ce que** l'ancrage sous-cutané est formé d'une pluralité de pattes de fixation (14) articulées sur le corps de base, les pattes de fixation pouvant pivoter à partir d'une position rétractée (E) radialement pliée vers une position de maintien (H) radialement dépliée, et **en ce que** les moyens de connexion inférieurs comportent une pièce d'adaptation (20) pour le tube cathéter qui peut être insérée dans le corps de base et qui coopère avec les pattes de fixation de telle manière que, dans une position insérée à butée d'arrêt inférieur, elle fixe les pattes de fixation en position de maintien radialement dépliée.

2. Dispositif de passage cutané selon la revendication 1, dans lequel les pattes de fixation (14) sont essentiellement en forme de O et ont des bords arrondis et, dans leur position rétractée (E), se trouvent dans un contour défini par le corps de base.

3. Dispositif de passage cutané selon la revendication 1 ou 2, dans lequel le corps de base est équipé dans la région inférieure d'un filetage intérieur (38) dans lequel peut être vissé un anneau fileté inférieur (40) pour maintenir la pièce d'adaptation dans sa position à butée d'arrêt inférieur.

4. Dispositif de passage cutané selon la revendication 3, dans lequel l'anneau fileté inférieur est doté d'une pluralité de rainures d'engagement internes (72) pour un outil de vissage (70) configuré de manière correspondante (70).

5. Dispositif de passage cutané selon l'une des revendications 1 à 4, dans lequel le corps de base est équipé dans sa région supérieure d'évidements (66) côté bord pour la fixation d'un anneau de support (76) ou d'un outil de maintien (74).

6. Dispositif de passage cutané selon l'une des revendications 1 à 5, dans lequel les moyens de connexion supérieurs comprennent un élément tendeur (42) qui peut être vissé dans le corps de base et qui présente une cavité destinée à recevoir de manière remplissante un corps de septum (44) et qui et délimitée par une surface inférieure (46) comportant au moins une ouverture traversante (48).

7. Dispositif de passage cutané selon la revendication 6, dans lequel les moyens de connexion supérieurs comprennent en outre une tête de connexion (50) qui peut être connectée au tube externe et qui peut être connectée au côté supérieur de l'élément tendeur (42), la tête de connexion et l'élément tendeur étant équipés d'éléments de couplage coopérants (52, 54).

8. Dispositif de passage cutané selon la revendication 7, dans lequel les éléments de couplage coopérants comprennent un évidement supérieur (52) dans l'élément tendeur et une saillie (54) associée dans la tête de connexion.

9. Dispositif de passage cutané selon l'une des revendications 1 à 8, dans lequel la pièce d'adaptation (20) des moyens de connexion inférieurs comprend une tête d'adaptation (28) avec une surface de base (32) et un évidement (34) pour insérer de manière étanche le tube cathéter, avec au moins un canal traversant (36) étant formé entre l'évidement et la surface de base supérieure.

10. Dispositif de passage cutané selon la revendication 9, dans lequel la tête d'adaptateur (28) est en forme de dôme de telle sorte que lorsque la pièce d'adaptation est insérée dans le corps de base jusqu'à la position à butée d'arrêt inférieur, une surface (26) de la tête d'adaptateur interagit avec des zones d'extrémité (24) de forme correspondante des pattes de fixation, tellement que celles-ci pivotent dans la position de maintien radialement dépliée.

11. Dispositif de passage cutané selon la revendication 10, dans lequel l'évidement (34) pour l'insertion étanche du tube de cathéter (4) est disposé latéralement sur la tête d'adaptateur (28).

12. Dispositif de passage cutané selon la revendication 11, dans lequel le canal de passage dans la tête d adapteur (28) est formé par au moins une, de préférence trois canule(s), chaque canule étant configurée comme suit:
- une extrémité inférieure de canule (56) dépasse de l'évidement latéral (34),
- une zone de canule centrale attenante (58) est guidée en arc de cercle à travers la tête d'adaptateur,
- une extrémité supérieure attenante de canule (60) dépasse de la surface de base.

13. Dispositif pour administrer et/ou prélever du fluide à un patient, comprenant un dispositif de passage cutané selon l'une des revendications précédentes, un tube cathéter (4) qui y est connecté et un tube externe (2) qui y est connecté, qui peut être connecté à une unité de traitement.

14. Dispositif selon la revendication 13, dans lequel les moyens de connexion supérieurs comprennent un élément tendeur (42) vissé dans le corps de base, dans la cavité duquel est inséré un corps de septum (44) de manière remplissante, le corps de septum présentant au moins un canal de septum continu (62) dans lequel, d'une part, une extrémité supérieure de canule (60) de la pièce adaptatrice et d'autre part une extrémité inférieure de tube (64) de la tête de raccordement (50) communiquant avec le tuyau externe (2 ) sont insérés.
